# EUROPEAN PATENT APPLICATION

(11) **EP 2 789 629 A1**
(43) Date of publication of application: **15.10.2014**
(21) Application number: 13163419.8
(22) Date of filing: 11.04.2013
(51) Int. Cl.: C07K 16/18, A61P 3/06

(54) **Method for the treatment of lysozomal lipid storage diseases**

(71) Applicant: Universiteit Maastricht, 6211 LK Maastricht (NL); Academisch Ziekenhuis Maastricht, 6229 HX Maastricht (NL)
(72) Inventor: The designation of the inventor has not yet been filed
(74) Representative: Habets, Winand

(57) **Abstract**

The invention is in the field of medical treatments. The invention in particular addresses the treatment of lysozomal lipid storage diseases such as Niemann-Pick disease type C1. More in particular, the invention relates to a method for treating, ameliorating of preventing inflammation in a lysozomal storage disease, wherein the level of anti-oxLDL antibodies is increased in a subject in need of such a treatment.

## Description

### Field of the invention

The invention is in the field of medical treatments. The invention in particular addresses the treatment of lysozomal lipid storage diseases such as Niemann-Pick disease type C1.

### Background of the invention

Although rare, Niemann-Pick disease type C1 (NPC1) is an extremely severe disease with the majority of patients dying between 10 and 25 years of age (Vanier MT. Niemann-Pick disease type C. Orphanet journal of rare diseases. 2010; 5:16). Moreover, clinical features of NPC are extremely heterogeneous and range from systemic (lung, spleen, lung) to neurological symptoms.

NPC1 is an inherited lysosomal lipid storage disease resulting from a deletion in the NPC1 gene, leading to impaired intracellular lipid transport and accumulation of unesterified cholesterol in lysosomes of various tissues (Parkinson-Lawrence EJ, Shandala T, Prodoehl M, Plew R, Borlace GN, Brooks DA. Lysosomal storage disease: revealing lysosomal function and physiology. Physiology (Bethesda). 2010;25(2):102-15). The NPC1 gene encodes a lysosomal membrane protein involved in the translocation of cholesterol from the lysosome to the cytoplasm.

One of the proposed mechanisms that contribute to NPC1 pathology is an inappropriate inflammatory response, mediated by dysregulated activation of macrophages (Maxfield FR, Tabas I. Role of cholesterol and lipid organization in disease. Nature. 2005;438(7068):612-21.). Cholesterol trapped in lysosomes of macrophages has been shown to be very resistant to mobilization into the cytoplasm.

Despite of the severity of NPC1 and the above progress in the understanding of molecular mechanisms underlying the disease, there is still no treatment available (Yancey PG, Jerome WG. Lysosomal cholesterol derived from mildly oxidized low density lipoprotein is resistant to efflux. Journal of lipid research. 2001;42(3):317-27).

### Summary of the invention

Surprisingly, we found that the oxLDL fraction of the lysosomal cholesterol in macrophages is the actual trigger for the inflammatory response. Ex vivo experiments showed that decreasing the lysosomal fraction of oxLDL in NPC1 macrophages reduced inflammation and inhibited disease progression.

The invention therefore relates to a method for treating, ameliorating of preventing inflammation in a lysozomal storage disease, wherein the level of anti-oxLDL antibodies is increased in a subject in need of such a treatment.

### Detailed description of the invention

We found that Niemann-Pick disease type C1 (NPC1) may be treated or ameliorated by raising (increasing) the level of antibodies against oxidized LDL (anti-oxLDL) in patients with NPC1.

Oxidation-specific epitopes present on oxLDL, such as the phosphorylcholine (PC) headgroup, are major targets of natural IgM antibodies (Shaw PX, Horkko S, Chang MK, Curtiss LK, Palinski W, Silverman GJ, et al. Natural antibodies with the T15 idiotype may act in atherosclerosis, apoptotic clearance, and protective immunity. The Journal of clinical investigation. 2000;105(12):1731-40). These antibodies are similar to the so-called T15 antibodies, which protect mice against Streptococcus pneumoniae infections because PC is also present in the capsular polysaccharide of the cell wall of this bacterium. If Streptococcus pneumoniae is used to immunize mice lacking the low-density lipoprotein receptor gene (Ldlr-/- mice), then this results in higher serum titers of anti-oxLDL IgM antibodies due to molecular mimicry (Binder CJ, Horkko S, Dewan A, Chang MK, Kieu EP, Goodyear CS, et al. Pneumococcal vaccination decreases atherosclerotic lesion formation: molecular mimicry between Streptococcus pneumoniae and oxidized LDL. Nature medicine. 2003;9(6):736-43).

Via bone marrow transplantation, we obtained a stable mouse strain with a mutation in the NPC gene in the hematopoietic cells. These mice are an accepted model for Niemann-Pick C disease (Loftus et al., Science (1997) 277: 232-235) The activity of the NPC gene in these mice was dramatically reduced, resulting in an accumulation of cholesterol in the lysosomes of their macrophages. This mouse strain is herein further referred to as NPC-negative mice.

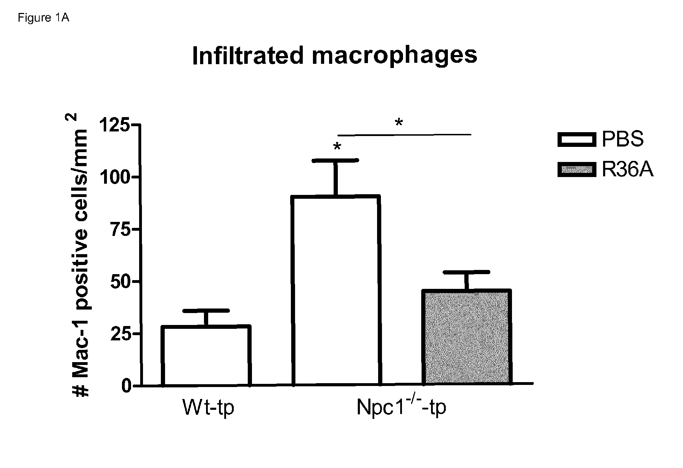

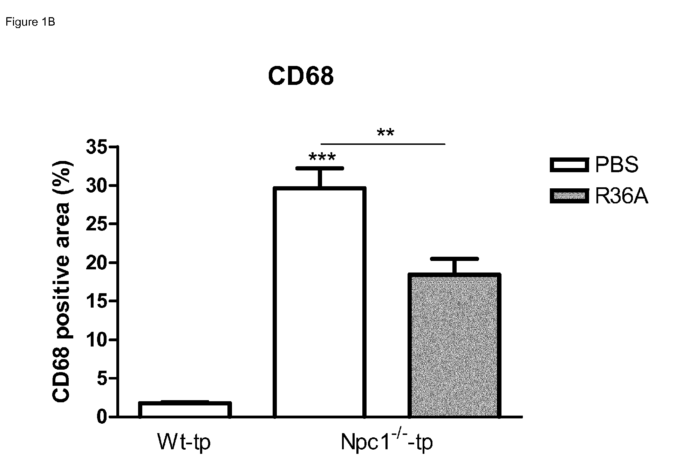

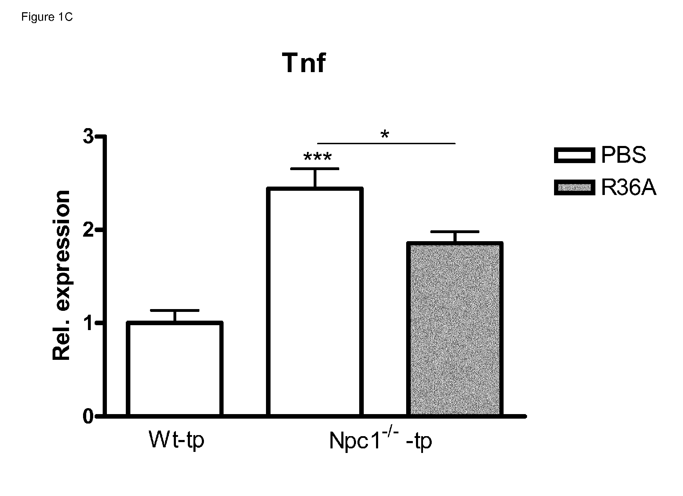

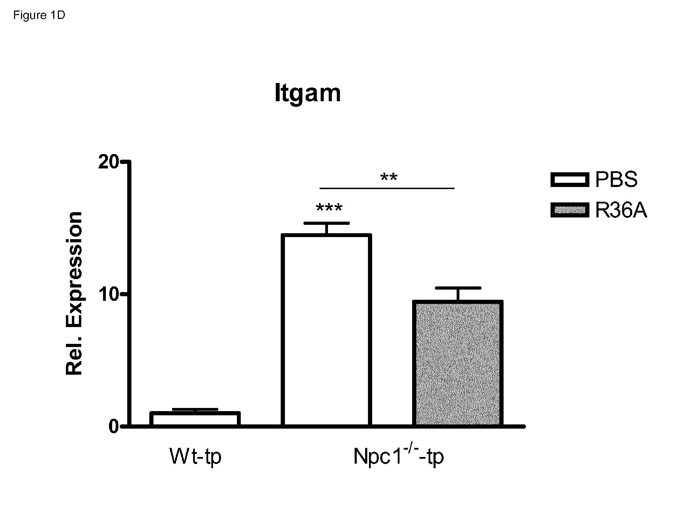

We herein present evidence that NPC-negative mice show decreased inflammation and a decreased cholesterol accumulation in macrophages after treatment with heat inactivated pneumococci (R36A). The results are shown in figure 1. These findings show that anti-oxLDL antibodies can reduce lysosomal cholesterol accumulation in macrophages of NPC-negative mice and decrease the associated inflammatory response.

The invention therewith provides a method for treating, ameliorating of preventing inflammation in a lysozomal storage disease, wherein the level of anti-oxLDL antibodies is increased in a subject in need of such a treatment.

In a preferred embodiment, the lysozomal storage disease is Niemann-Pick disease type C1 (NPC1).

The level of anti-oxLDL antibodies in a subject may be increased in a number of ways, known in the art *per se.* For example, the antibodies may be administered to the subject intravenously. In a particular embodiment, the invention therefore relates to a method as described above wherein the level of anti-oxLDL antibodies is increased by administering a composition comprising anti-oxLDL antibodies. In an alternative or supplementary way, the level of anti-oxLDL antibodies may be increased by administering a composition to the subject that comprises a PC-headgroup. The invention therefore also relates to a method as described above wherein the level of anti-oxLDL antibodies is increased by administering a composition comprising a PC headgroup. A particularly preferred composition comprising a PC-headgroup is a composition comprising a Streptococcus pneumoniae antigen, such as a Streptococcus pneumoniae antigen derived from heat-inactivated Streptococcus pneumoniae.

### Legend to the figures

Figure 1: Treatment with heat inactivated pneumococci (R36A) decreases inflammation and cholesterol accumulation in macrophages from NPC-negative mice (NPC-1^{-/-} -tp), a murine model for Niemann-Pick disease.
   Panels A and B: Livers from NPC-negative mice treated with R36A show decreased infiltration of macrophages and CD68 positive area compared to control treated NPC-negative mice. While an increased level of infiltrated macrophages gives an indication of the level of inflammation, the CD68 positive area assesses the amount of lipids stored inside the macrophages.
   Panels C and D: Gene expression levels of inflammatory markers tumor necrosis factor (Tnf) and integrin alpha M (ltgam) are reduced in livers of NPC-negative (Npc1^{-/-}-tp) mice treated with R36A compared to control treated Npc1^{-/-}-tp mice. *,**,*** indicate p<0.05, 0.01 and 0.001 respectively.

### EXAMPLES

### Example 1: Lysosomal oxLDL contributes to disease progression in NPC1

We investigated the effect of anti-oxLDL antibodies on inflammation in vitro by loading macrophages from NPC-negative mice with control, LDL or oxLDL and incubate them with or without the anti-ox-LDL antibodies. Analysis of inflammation involved gene expression and protein analysis (qPCR, ELISA, Western Blot).

Also, the same experiments may be performed on macrophages of NPC1 patients (ex vivo).

We found that oxLDL-loaded macrophages from NPC-negative mice treated with anti-oxLDL antibodies showed decreased inflammation compared to macrophages without treatment. Furthermore, we also found that non-treated macrophages from NPC-negative mice showed increased inflammation when loaded with oxLDL compared to LDL- or control-loaded macrophages. These in vitro and ex vivo experiments allowed us to conclude that the oxLDL fraction of lysosomal cholesterol contributed to disease progression of NPC1 and that antibodies against oxLDL could be used as an effective therapy in lysosomal lipid storage diseases such as Niemann-Pick type C disease..

### Example 2: Reducing lysosomal oxLDL levels in NPC-negative mice inhibits disease progression

The following experiments may be performed on three week-old NPC-negative mice. Disease progression of NPC-negative mice may be determined in mice receiving R36A injections with NaCl as a control, given every two weeks intraperitoneally.

Progression of NPC1 may be determined by assessing survival rate and analysis of disease severity (i.e. amount of lysosomal cholesterol). All metabolic tissues (liver, spleen, gut, arterial wall) may be analyzed. Additionally, the brain may also be examined. Analysis of tissues may involve biochemical determination of lipids, blood analysis, histology, electron microscopy, qPCR and protein analysis.

The treatment with anti-ox-LDL antibodies will then be shown to decrease disease severity and to increase the life span.

## Claims

1. Anti-oxLDL antibodies for use in treating, ameliorating of preventing inflammation in a lysozomal storage disease, wherein the level of anti-oxLDL antibodies is increased in a subject in need of such a treatment.

2. Anti-oxLDL antibodies for use according to claim 1 wherein the lysozomal storage disease is Niemann-Pick disease type C1 (NPC1).

3. Anti-oxLDL antibodies for use according to claims 1 or 2 wherein the level of anti-oxLDL antibodies is increased by administering a composition comprising anti-oxLDL antibodies.

4. Anti-oxLDL antibodies for use according to claims 1 or 2 wherein the level of anti-oxLDL antibodies is increased by administering a composition comprising a PC headgroup.

5. Anti-oxLDL antibodies for use according to claim 4 wherein the composition comprising a PC headgroup is a Streptococcus pneumoniae antigen.

6. Anti-oxLDL antibodies for use according to claim 5 wherein the Streptococcus pneumoniae antigen is derived from heat-inactivated Streptococcus pneumoniae.

7. Method for treating, ameliorating of preventing inflammation in a lysozomal storage disease, wherein the level of anti-oxLDL antibodies is increased in a subject in need of such a treatment.

8. Method according to claim 6 wherein the lysozomal storage disease is Niemann-Pick disease type C1 (NPC1).

9. Method according to claims 6 or 8 wherein the level of anti-oxLDL antibodies is increased by administering a composition comprising anti-oxLDL antibodies.

10. Method according to claims 7 or 8 wherein the level of anti-oxLDL antibodies is increased by administering a composition comprising a PC headgroup.

11. Method according to claim 10 wherein the composition comprising a PC headgroup is a Streptococcus pneumoniae antigen.

12. Method according to claim 11 wherein the Streptococcus pneumoniae antigen is derived from heat-inactivated Streptococcus pneumoniae.
